(19)

Europäisches Patentamt
European Patent Office
Office européen des brevets

(11) **EP 1 961 384 A1**

(12) **EUROPEAN PATENT APPLICATION**
published in accordance with Art. 158(3) EPC

(43) Date of publication:
**27.08.2008 Bulletin 2008/35**

(51) Int Cl.:
**A61B 8/08** (2006.01)

(21) Application number: **06834610.5**

(22) Date of filing: **13.12.2006**

(86) International application number:
**PCT/JP2006/324855**

(87) International publication number:
**WO 2007/069650 (21.06.2007 Gazette 2007/25)**

(84) Designated Contracting States:
**DE GB**

(30) Priority: **15.12.2005 JP 2005362057**

(71) Applicants:
• **Matsushita Electric Industrial Co., Ltd.**
**Kadoma-shi**
**Osaka 571-8501 (JP)**
• **TOHOKU UNIVERSITY**
**Aoba-ku**
**Sendai-shi, Miyagi 980-8577 (JP)**

(72) Inventors:
• **SUZUKI, Takao**
**c/o Matsushita Electric Industrial Co., Ltd.**
**Intellectual Property Rights Operations Company**
**Osaka 540-6207 (JP)**
• **HAGIWARA, Hisashi**
**c/o Matsushita Electric Industrial Co., Ltd.**
**Intellectual Property Rights Operations Company**
**Osaka 540-6207 (JP)**

• **KATO, Makoto**
**c/o Matsushita Electric Industrial Co., Ltd.**
**Intellectual Property Rights Operations Company**
**Osaka 540-6207 (JP)**
• **TAN-NAKA, Yoshinao**
**c/o Matsushita Electric Industrial Co., Ltd.**
**Intellectual Property Rights Operations Company**
**Osaka 540-6207 (JP)**
• **KANAI, Hiroshi**
**Sendai-shi**
**Miyagi 980-8577 (JP)**
• **HASEGAWA, Hideyuki**
**Sendai-shi**
**Migayi 980-8577 (JP)**

(74) Representative: **Grünecker, Kinkeldey,**
**Stockmair & Schwanhäusser**
**Anwaltssozietät**
**Leopoldstrasse 4**
**80802 München (DE)**

(54) **ULTRASONOGRAPH**

(57) An ultrasonic diagnostic apparatus according to the present invention is designed to measure a vascular wall. The apparatus includes: a transmitting section that drives a probe to transmit an ultrasonic wave toward a subject including a blood vessel; a receiving section that receives an ultrasonic echo, produced by getting the ultrasonic wave reflected by the subject, at the probe to generate a received signal; a tomographic image processing section for generating an image signal based on the received signal to present the tomographic image of the subject on a display section; a first borderline generating section for determining a first type of borderline based on the tomographic image presented on the display section; and a second borderline generating section for generating at least one borderline of a second type by translating the first type of borderline.

FIG.4

**Description**

TECHNICAL FIELD

The present invention relates to an ultrasonic diagnostic apparatus for use in medical applications and more particularly relates to an ultrasonic diagnostic apparatus for measuring a vascular wall.

BACKGROUND ART

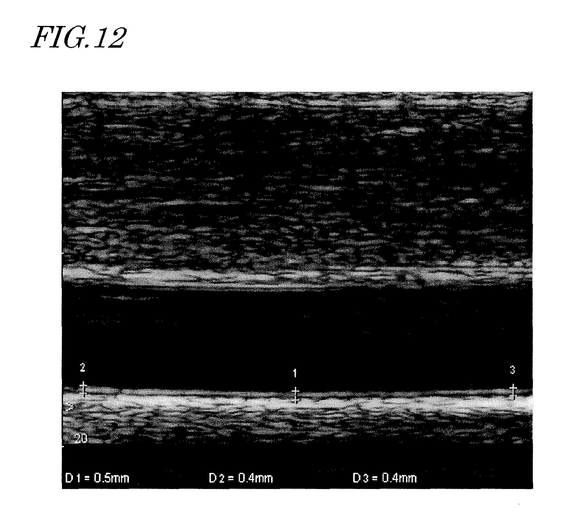

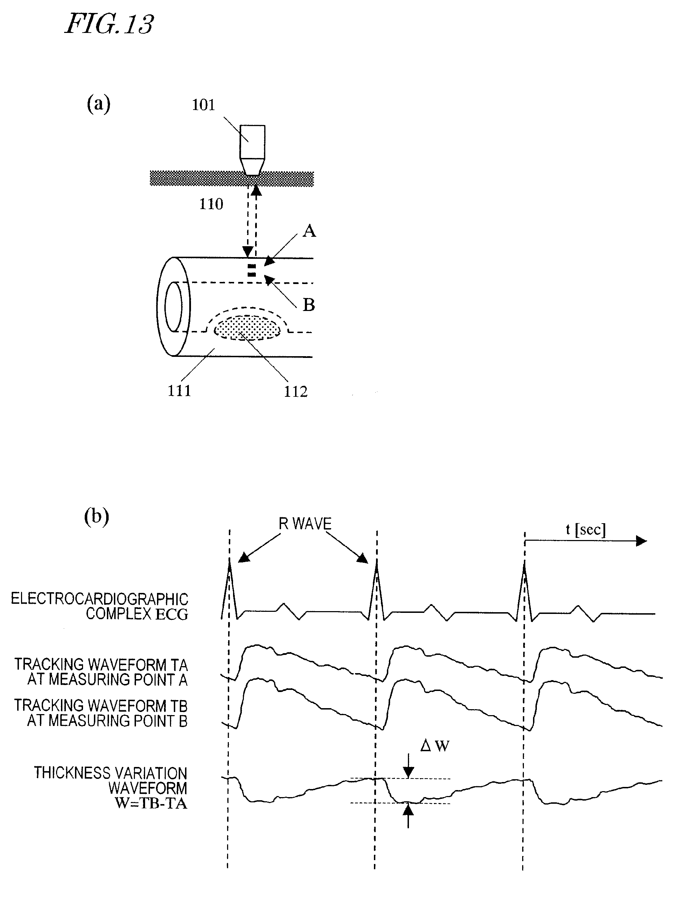

An ultrasonic diagnostic apparatus is used to monitor an internal tissue of a subject by irradiating him or her with an ultrasonic wave and analyzing the information contained in its echo signal. For example, a conventional ultrasonic diagnostic apparatus that has been used extensively converts the intensity of an echo signal into its associated pixel luminance, thereby presenting the subject's structure as a tomographic image. In this manner, the internal structure of the subject can be known. The ultrasonic diagnostic apparatus can be used to make a noninvasive checkup on an internal tissue of a subject, and therefore, is now an indispensable device at any clinical spot along with X-ray CT and MRI.
Recently, the number of people suffering from arteriosclerosis has been on the rise and carotid echo has been carried out more and more often using an ultrasonic diagnostic apparatus to diagnose arteriosclerosis. It is known that the carotid artery has a three-layer structure consisting of intima, media and adventitia that are stacked in this order. In carrying out a carotid echo, the combined thickness of the intima and the media (or intima-media thickness, which will be abbreviated herein as "IMT") is measured and used as an index to arteriosclerosis. According to Non-Patent Document No. 1, if the IMT is 1.1 mm or more, the carotid is determined to have abnormally thickened. The IMT is manually measured with a length measuring function on a tomographic image, which is usually provided for any ultrasonic diagnostic apparatus. The IMT may be measured sometimes at only one spot and sometimes at multiple spots. In the latter case, the maximum or average of those multiple measured values may be used as an index. FIG. **12** shows how the IMT of a carotid artery is measured with a conventional ultrasonic diagnostic apparatus. Specifically, FIG. **12** shows a tomographic image as viewed on a plane that is parallel to the axis of the vascular wall (i.e., a vertical cross section of the vascular wall). In FIG. **12,** the six + signs indicate manually set points. At the central pair of set points indicated by "1", the distance between the two + signs is 0.5 mm. On the other hand, at each of the other two pairs of set points indicated by "2" and "3", the distance between the two + signs is 0.4 mm.
Meanwhile, as an alternative method for diagnosing arteriosclerosis, some people are attempting recently to track the motion of a subject's tissue more precisely and evaluate the strain and the elasticity, viscosity or any other attribute property of the tissue mainly by analyzing the phase of the reflected echo signal.
Patent Document No. 1 discloses a method for tracking precisely the motions of two very small regions on a vascular wall in one cardiac cycle, sensing a slight variation in the thickness (i.e., the magnitude of strain) that is superposed on a significant oscillation due to the heartbeat and calculating a local elasticity based on the magnitude of strain and blood pressure difference. Patent Document No. 1 also discloses an apparatus for displaying a spatial distribution of elasticities as an image. To track those very small regions on a vascular wall, a phase difference tracking method as disclosed in Patent Document No. 2 is used. Hereinafter, a method for tracking a subject's tissue as disclosed in Patent Document No. 1 will be described with reference to FIGS. **13(a)** and **13(b).** As shown in FIG. **13(a),** an ultrasonic wave is transmitted from a probe **101** toward the blood vessel **111** of a subject **110** and an echo reflected from the blood vessel **111** is received at the probe **101.** Two measuring points **A** and **B** are set on the vascular wall and the signals received from those measuring points **A** and **B** are analyzed by the method disclosed in Patent Document No. 2, thereby tracking the motions of the measuring points **A** and **B.**
The blood vessel **111** repeatedly contracts and dilates through cardiac cycles. More specifically, the blood vessel **111** dilates rapidly in a systolic phase but contracts slowly in a diastolic phase. FIG. **13(b)** shows the tracking waveforms **TA** and **TB** of the measuring points **A** and **B** along with an electrocardiographic complex **ECG.** As the blood vessel **111** dilates, the measuring points **A** and **B** move rapidly. But after that, the measuring points **A** and **B** slowly go back to their original locations. The difference between the tracking waveforms **TA** and **TB** is represented as a waveform **W** showing a variation in thickness between the measuring points **A** and **B.** Supposing the variation in the thickness variation waveform is ∆M and the reference thickness between the measuring points during initialization is Ws, the magnitude of strain ε between the measuring points **A** and **B** is calculated by the following Equation (1):
$$\varepsilon = \Delta W / W_S \qquad (1)$$

Supposing the blood pressure difference at this time is ∆P, the radial elasticity **Er** between the measuring points **A** and **B** is given by the following Equation (2):
$$Er = \Delta P / \varepsilon = \Delta P \cdot Ws / \Delta W \qquad (2)$$

Therefore, by measuring the elasticity **Er** for multiple spots on a tomographic image, an image representing the distribution of elasticities can be obtained.

Non-Patent Document No. 2 discloses a method for calculating the circumferential elasticity of a blood vessel, representing a more accurate physical property than the radial elasticity thereof. According to Non-Patent Document No. 2, the circumferential elasticity is given by the following Equation (3):

$$E\theta = -(1/2) \cdot (r0/h0 + 1) \cdot (\Delta P / \varepsilon)$$

$$= -(1/2) \cdot (r0/h0 + 1) \cdot Er \qquad (3)$$

where h0 is the initial radial thickness of the overall vascular wall and r0 is initial radius of the blood vessel.

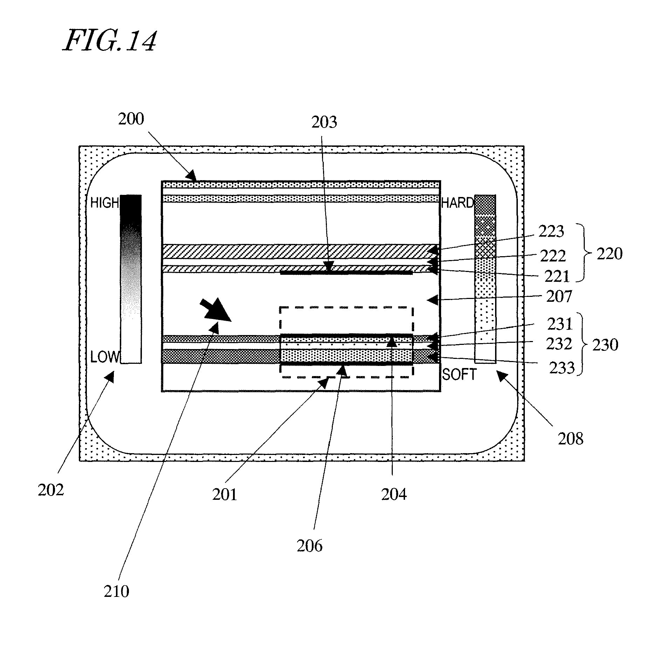

FIG. **14** illustrates a picture to be presented on a monitor screen when the artery is inspected with an ultrasonic diagnostic apparatus. On the screen, an elasticity image **201** representing the distribution of elasticities is superimposed on the posterior wall portion **230** (i.e., a portion of the vascular wall that is more distant from the skin surface) of a tomographic image **200.**

In the prior art, to calculate the initial thickness **h0** and initial radius **r0** of the overall vascular wall, the borderline **203** between the vascular flow **207** and the intima **221** of the anterior wall (i.e., a portion of the vascular wall closer to the skin surface) **220,** the borderline **204** between the vascular flow **207** and the intima **231** of the posterior wall **230,** and the borderline **206** between the adventitia **233** of the posterior wall **230** and its surrounding tissue are drawn manually. More specifically, by sensing the boundary between the area of the vascular flow **207** and the area of the intima **221** of the anterior wall **220** that are presented on the monitor screen based on the shades of the tomographic image reflection intensity scale **202,** the operator traces the borderline with the cursor **210** on the monitor screen, thereby determining the borderline **203**. By repeatedly performing similar operations, the other borderlines **204** and **206** can also be drawn.

**Patent Document No. 1:** Japanese Patent Application Laid-Open Publication No. 2000-229078
**Patent Document No. 2:** Japanese Patent Application Laid-Open Publication No. 10-5226
**Non-Patent Document No. 1:** Hiroshi Furuhata, Carotid Echo, Vector Core Inc., 2004, ISBN 4-938372-88-6
**Non-Patent Document No. 2:** Hideyuki Hasegawa, Hiroshi Kanai et al., "Evaluation of regional elastic modulus of cylindrical shell with non-uniform wall thickness", J. Med. Ultrasonics, Vol. 28, No. 1 (2001), pp. J3 to J13

## DISCLOSURE OF INVENTION

## PROBLEMS TO BE SOLVED BY THE INVENTION

However, as there are multiple borderlines to draw, it will take a while to establish all of those borderlines. Also, since the initial thickness **h0** and initial radius **r0** of the overall vascular wall are calculated based on the borderlines that have been drawn in this manner, the borderlines should be determined accurately. For that reason, it would impose a heavy burden on the operator to determine the borderlines by moving the cursor by him- or herself.

In order to overcome the problems described above, the present invention has an object of providing an ultrasonic diagnostic apparatus that can alleviate the burden on the operator.

## MEANS FOR SOLVING THE PROBLEMS

An ultrasonic diagnostic apparatus according to the present invention is designed to measure a vascular wall. The apparatus includes: a transmitting section that drives a probe to transmit an ultrasonic wave toward a subject including a blood vessel; a receiving section that receives an ultrasonic echo, produced by getting the ultrasonic wave reflected by the subject, at the probe to generate a received signal; a tomographic image processing section for generating an image signal based on the received signal to present the tomographic image of the subject on a display section; a first borderline generating section for determining a first type of borderline based on the tomographic image presented on the display section; and a second borderline generating section for generating at least one borderline of a second type by translating the first type of borderline.

In one preferred embodiment, the ultrasonic diagnostic apparatus further includes a user interface that allows an operator to specify his or her desired location on the tomographic image.

In this particular preferred embodiment, the borderline generating section generates the second type of borderline by translating the first type of borderline to the location that has been specified by the operator with the user interface in accordance with information about the first type of borderline.

In a specific preferred embodiment, the first borderline generating section stores, as the information about the first type of borderline, information about a line segment that has been drawn on the tomographic image by the operator with the user interface.

In a more specific preferred embodiment, the first borderline generating section generates either a line segment or a polygon as the first type of borderline at the location that has been specified by the operator.

In another preferred embodiment, the first type of borderline is located on at least one boundary that is selected from a group of boundaries consisting of boundaries between the intima of the anterior and posterior walls of the blood vessel and vascular flow, boundaries between the media and adventitia of the anterior and posterior walls of the blood vessel, and boundaries between the adventitia of the anterior and posterior walls of the blood vessel and their surrounding tissue. The second type of borderline is located on at least another one of the group of boundaries.

In this particular preferred embodiment, the first borderline generating section detects the boundary between the intima of the anterior or posterior wall of the blood vessel and the vascular flow based on the image signal and determines the first type of borderline on the boundary detected.

In a specific preferred embodiment, the first type of borderline is located on the boundary between the intima of the anterior or posterior wall and the vascular flow and the second type of borderline is located on the boundary between the media and adventitia thereof. The intima-media thickness of the vascular wall is calculated by reference to the first and second types of borderlines.

In a more specific preferred embodiment, the ultrasonic diagnostic apparatus further includes: a tissue tracking section for tracking the motion of a tissue of the subject based on the received signal; and an attribute value calculating section that receives information about the blood pressure of the subject and calculates an attribute value of the tissue based on the motion of the tissue tracked. The attribute value of at least one tissue, which is selected from the group consisting of the intima, media and adventitia of the blood vessel, is calculated with respect to the first and second types of borderlines.

In this particular preferred embodiment, the first type of borderline is located on the boundary between the intima and the vascular flow and the second type of borderline is located between the adventitia and the surrounding tissue. By calculating the radius of the blood vessel and the thickness of the vascular wall by reference to the first and second types of borderlines, a circumferential elasticity is calculated as the attribute value.

A method for determining the boundaries of a vascular wall using an ultrasonic diagnostic apparatus according to the present invention includes the step of generating a second type of borderline by translating a first type of borderline on a tomographic image of a subject's blood vessel that has been produced by transmitting and receiving an ultrasonic wave. The first type of borderline is determined so as to be located on at least one boundary that is selected from a group of boundaries consisting of boundaries between the intima of the anterior and posterior walls of the blood vessel and vascular flow, boundaries between the media and adventitia of the anterior and posterior walls of the blood vessel, and boundaries between the adventitia of the anterior and posterior walls of the blood vessel and their surrounding tissue. The second type of borderline is located on at least another one of the group of boundaries.

In one preferred embodiment, the method further includes the step of storing, as information about the first type of borderline, information about a line segment that has been drawn on the tomographic image by the operator using a user interface. The step of generating the second type of borderline includes generating the second type of borderline based on the stored information about the first type of borderline.

In another preferred embodiment, the method further includes the step of generating either a line segment or a polygon as the first type of borderline at the location that has been specified on the tomographic image of the blood vessel by the operator using the user interface.

In still another preferred embodiment, the method further includes the step of detecting the boundary between the intima of the anterior or posterior wall of the blood vessel and the vascular flow based on an image signal representing the tomographic image and determining the first type of borderline on the boundary detected.

## EFFECTS OF THE INVENTION

According to the present invention, time and trouble to determine the respective boundaries of a vascular wall can be saved and measurements by an ultrasonic diagnostic apparatus can be done in a shorter time.

## BRIEF DESCRIPTION OF DRAWINGS

FIG. **1(a)** is a block diagram illustrating a configuration for an ultrasonic diagnostic apparatus according to the present invention and FIG. **1(b)** is a block diagram illustrating the configuration of its core section.
FIG. **2** is a flowchart showing a procedure for determining borderlines using the ultrasonic diagnostic apparatus shown in FIG. **1.**
FIG. **3** illustrates an exemplary picture to be presented on the monitor screen when the procedure shown in FIG. **2** is adopted.
FIGS. **4(a)** through **4(c)** illustrate an exemplary procedure for generating a borderline by translating another borderline.
FIGS. **5(a)** through **5(c)** illustrate another exemplary procedure for generating a borderline by translating another borderline.
FIGS. **6(a)** through **6(c)** illustrate still another exemplary procedure for generating a borderline by translating another borderline.
FIG. **7** is a flowchart showing another procedure for determining borderlines using the ultrasonic diagnostic apparatus shown in FIG. **1.**
FIG. **8** illustrates an exemplary picture to be presented on the monitor screen when the procedure shown in FIG. **7** is adopted.
FIG. **9** is a flowchart showing still another procedure for determining borderlines using the ultrasonic diagnostic apparatus shown in FIG. **1.**
FIG. **10** illustrates an exemplary picture to be presented on the monitor screen when the procedure shown in FIG. **8** is adopted.
FIG. **11** is a flowchart showing yet another procedure for determining borderlines using the ultrasonic diagnostic apparatus shown in FIG. **1.**
FIG. **12** shows an exemplary plane on which an IMT is measured with a conventional ultrasonic diagnostic apparatus.
FIG. **13(a)** illustrates a procedure for calculating the magnitude of strain based on the tracking waveforms at multiple measuring points using a conventional ultrasonic diagnostic apparatus and FIG. **13(b)** shows the tracking waveforms at those measuring points.
FIG. **14** illustrates a typical elasticity image presented on the screen of a conventional ultrasonic diagnostic apparatus.

## DESCRIPTION OF REFERENCE NUMERALS

| | |
|---|---|
| **100** | control section |
| **101** | probe |
| **102** | transmitting section |
| **103** | receiving section |
| **104** | tomographic image processing section |
| **105** | tissue tracking section |
| **106** | image synthesizing section |
| **107** | monitor |
| **108** | elasticity calculating section |
| **111** | blood pressure manometer |
| **112** | blood pressure manometer controlling and blood pressure value input section |
| **120, 121** | memory |
| **130** | user interface |
| **150** | borderline determining section |
| **151** | borderline storage section |
| **152** | second borderline generating section |
| **153** | first borderline generating section |
| **200** | tomographic image |
| **202** | tomographic image reflection intensity scale |
| **203** | vascular flow-intima borderline on anterior wall of blood vessel |

204    vascular flow-intima borderline on posterior wall of blood    vessel
205    media-adventitia borderline on posterior wall of blood vessel
206    adventitia-surrounding tissue borderline on posterior wall    of blood vessel

## BEST MODE FOR CARRYING OUT THE INVENTION

Hereinafter, preferred embodiments of an ultrasonic diagnostic apparatus according to the present invention will be described with reference to the accompanying drawings. An ultrasonic diagnostic apparatus according to the present invention is used to determine the shape or an attribute value of a vascular wall. In the preferred embodiments to be described below, a boundary between the vascular flow and the intima of the anterior wall of a blood vessel and boundaries between the vascular flow and the intima, between the media and adventitia, and between the adventitia and a surrounding tissue of the posterior wall of a blood vessel are supposed to be determined. However, the present invention is in no way limited to those specific preferred embodiments but is also applicable for use to determine a boundary between the media and adventitia of the anterior wall and a boundary between the adventitia and a surrounding tissue of the anterior wall.

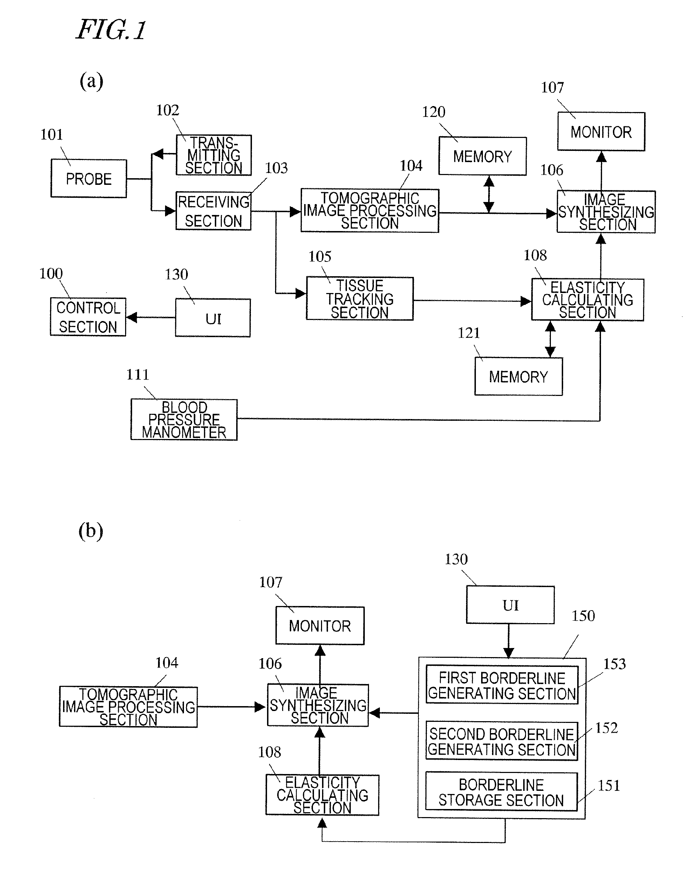

FIG. **1(a)** is a block diagram illustrating a configuration for an ultrasonic diagnostic apparatus as a preferred embodiment of the present invention. The ultrasonic diagnostic apparatus includes a transmitting section **102,** a receiving section **103,** a tomographic image processing section **104,** a tissue tracking section **105,** an image synthesizing section **106,** and an elasticity calculating section **108.** The apparatus further includes a control section **100** for controlling all of these components and a user interface **130.**

The user interface **130** is an input device that accepts an operator's command such as a keyboard, a trackball, a switch or a button. The operator's command that has been entered through the user interface **130** is input to the control section **100.** The control section **100** may be implemented as a microcomputer, for example, and controls all of those components in accordance with the operator's commands. In addition, as will be described in detail later with reference to FIG. **1(b),** the control section **100** includes a borderline determining section **150** for determining borderlines indicating boundaries between the tissues of a vascular wall. It should be noted that although the control section **100** actually exchanges signals with the respective blocks shown in FIG. **1(a),** lines indicating exchange of those signals are not shown in FIG. **1(a)** to avoid complications.

In accordance with the instruction given by the control section **100,** the transmitting section **102** generates a high-voltage transmission signal that drives the probe **101** at a specified timing. The probe **101** converts the transmission signal that has been generated by the transmitting section **102** into an ultrasonic wave and sends out the ultrasonic wave toward a subject, and also detects an ultrasonic echo that has been reflected by an internal organ of the subject and converts the echo into an electrical signal. A number of piezoelectric transducers are arranged in the probe **101.** By changing the piezoelectric transducers to use and the timing to apply a voltage to the piezoelectric transducers, the probe **101** controls the angle of deviation and focus of the ultrasonic waves to transmit and receive. The receiving section **103** amplifies the electrical signal generated by the probe **101** and outputs a received signal. Also, the receiving section **103** detects only an ultrasonic wave that has come from a particular position (associated with the focus) or direction (associated with the angle of deviation).

The tomographic image processing section **104** includes a filter, a detector, and a logarithmic amplifier, and analyzes mainly the amplitude of the received signal, thereby generating an image signal representing a tomographic image of the subject. The tissue tracking section **105** includes a magnitude of displacement calculating section for analyzing the phase difference between the received signals and calculating the magnitude of displacement of the subject's tissue in the ultrasonic wave transmitting and receiving directions, and a tracking location calculating section that calculates the new location by adding the magnitude of displacement to the original location. The tissue tracking section **105** tracks the motion of the subject's tissue in the ultrasonic wave transmitting and receiving directions.

The elasticity calculating section **108** calculates an attribute property value such as the magnitude of strain or the elasticity. Specifically, first, the elasticity calculating section **108** calculates the magnitude of strain based on the motion of the subject's tissue tracked. The elasticity calculating section **108** also receives information about the blood pressure of the subject from the blood pressure manometer **111.** Furthermore, as will be described in detail later, the elasticity calculating section **108** receives information about first and second types of borderlines that have been determined by the borderline determining section **150,** calculates the distance between the borderlines in the subject, and also calculates the radius of the blood vessel and the thickness of the vascular wall. And based on the magnitude of strain, the information about the blood pressure, the radius of the blood vessel, and the thickness of the vascular wall, the elasticity calculating section **108** calculates the radial and circumferential elasticities and outputs them as numerical values or a two-dimensional distribution image.

The image synthesizing section **106** synthesizes together the tomographic image and at least one of the elasticity image and the elasticity values and outputs the synthesized image to the monitor **107.** The memory **121** stores the location tracking information (i.e., the motion of the subject's tissue) and/or the magnitude of strain. With the transmission and reception of the ultrasonic waves by the probe **101** suspended (which will be referred to herein as a "freeze state"), the information stored in the memory **121** is read to re-calculate the elasticity values. On the other hand, the memory **120** stores the image signal. In the freeze state, the image signal representing the tomographic image is read synchronously with the elasticity.

Next, the borderline determining section **150** will be described in detail. FIG. **1(b)** is a block diagram illustrating the core section of the present invention. The borderline determining section **150** draws a first type of borderline based on the tomographic image presented on the monitor **107** and then generates at least one borderline of a second type by translating the first type of borderline thus determined.

For that purpose, the borderline determining section **150** includes a first borderline generating section **153,** a second borderline generating section **152** and a borderline storage section **151.** The first borderline generating section **153** draws a first type of borderline based on the tomographic image and stores information about the first type of borderline in the borderline storage section **151.** This first type of borderline is a line segment to be drawn as a cursor trace by allowing the operator to move the cursor using the user interface **130** such as a mouse while the tomographic image of the subject is being presented on the monitor **107.**

The first borderline generating section **153** may regard either a line segment or a polygon, which connects two or more points that have been specified by the operator with the mouse clicked, as the first type of borderline and store its information. Alternatively, the first borderline generating section **153** may obtain a tropic by connecting a plurality of points specified by the operator and regard the tropic as the first type of borderline. Still alternatively, the first borderline generating section **153** may also obtain a spline function that passes a plurality of points specified by the operator and use the spline function as the first type of borderline. The number of borderlines of the first type does not have to be one. If necessary, multiple borderlines may be drawn as borderlines of the first type.

In accordance with the information about the first type of borderline that is stored in the borderline storage section **151,** the second borderline generating section **152** translates the first type of borderline to the location specified by the operator, thereby generating a second type of borderline. Optionally, if multiple borderlines of a first group have been determined and stored by the first borderline generating section **153,** then the user interface **130** may select one of those borderlines of the first group stored.

The information about the first and second types of borderlines is output to the elasticity calculating section **108.** Based on these pieces of information, the elasticity calculating section **108** figures out the distance between the borderlines in the subject and uses the distance thus obtained to calculate the elasticity.

Such functions of the borderline determining section **150** will be described more fully with reference to a tomographic image presented on the monitor **107.**

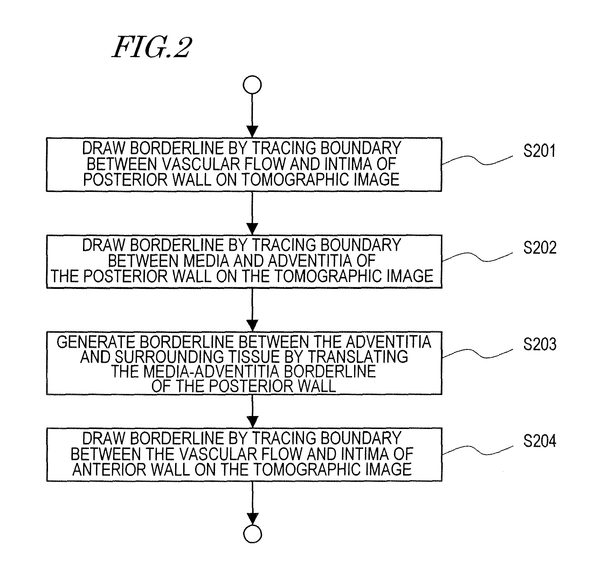

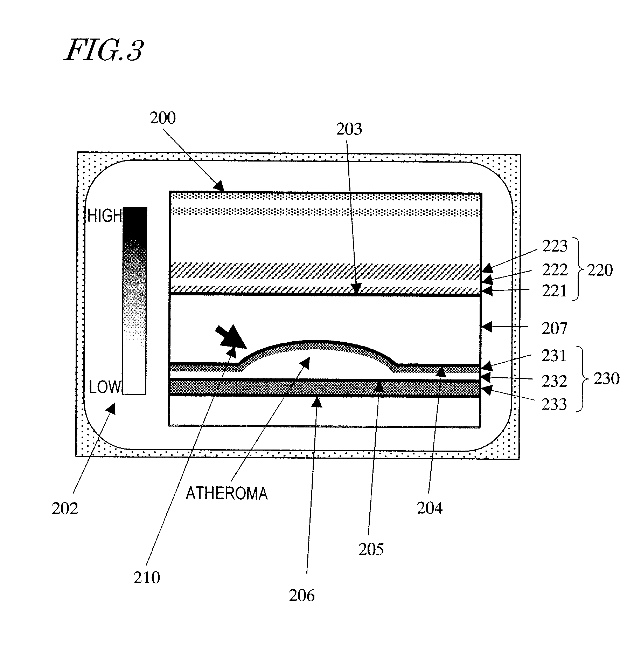

FIG. **2** is a flowchart showing the procedure of determining borderlines between the respective tissues of a vascular wall using a tomographic image that is viewed on a plane parallel to the axis of the blood vessel. First, the ultrasonic diagnostic apparatus transmits and receives ultrasonic waves to/from an internal organ of a subject, thereby getting a tomographic image of his or her vascular wall. As shown in FIG. **3,** a tomographic image **200** that has been obtained properly shows clearly intima **221,** media **222** and adventitia **223** in the anterior wall **220** and another intima **231,** another media **232** and another adventitia **233** in the posterior wall **230.** FIG. **3** shows a case where a boss called "atheroma" has been produced between the intima **231** and media **232** of the posterior wall **230** due to the congestion of fat or cholesterol there. To determine the borderlines between the respective tissues of the vascular wall in the state shown in this tomographic image **200,** the ultrasonic diagnostic apparatus is made to enter the freeze state.

First, in Step **S201,** by moving a cursor **210,** which is displayed on the screen of the monitor **107,** using the user interface **130** such as a trackball or a mouse provided for the ultrasonic diagnostic apparatus, the boundary between the vascular flow **207** and the intima **231** of the posterior wall **230** on the tomographic image **200** is traced with the cursor **210.** As a result, a line segment is drawn as a borderline **204** between the vascular flow **207** and the intima **231** of the posterior wall **230** on the screen. The borderline storage section **151** stores information about the borderline **204** as a first type of borderline.

Next, in Step **S202,** a borderline **205** is determined in a similar manner between the media **232** and the adventitia **233** of the posterior wall **230.** The borderline storage section **151** stores information about the borderline **205** as another borderline of the first type.

Subsequently, in Step **S203,** the second borderline generating section **152** translates the borderline **205** to the location specified by the operator in accordance with the information about the borderline **205** that is stored in the borderline storage section **151,** thereby generating a borderline **206** on the boundary between the adventitia **233** of the posterior wall **230** and its surrounding tissue.

Finally, in Step **S204,** by moving the cursor **210** on the screen of the monitor **107,** the boundary between the vascular flow **207** and the intima **221** of the anterior wall **220** on the tomographic image **200** is traced with the cursor **210,** thereby drawing a borderline **203** between the vascular flow **207** and the intima **221** of the anterior wall **220.** The borderline storage section **151** stores information about the borderline **203** as another borderline of the first type. By following such a procedure, the burden imposed on the operator by tracing boundaries and the time it takes to determine the borderlines can be reduced by one step.

In the processing step **S203,** the borderline can be generated by any of various image processing techniques such as copying a line segment on a computer screen and pasting it at a predetermined location.

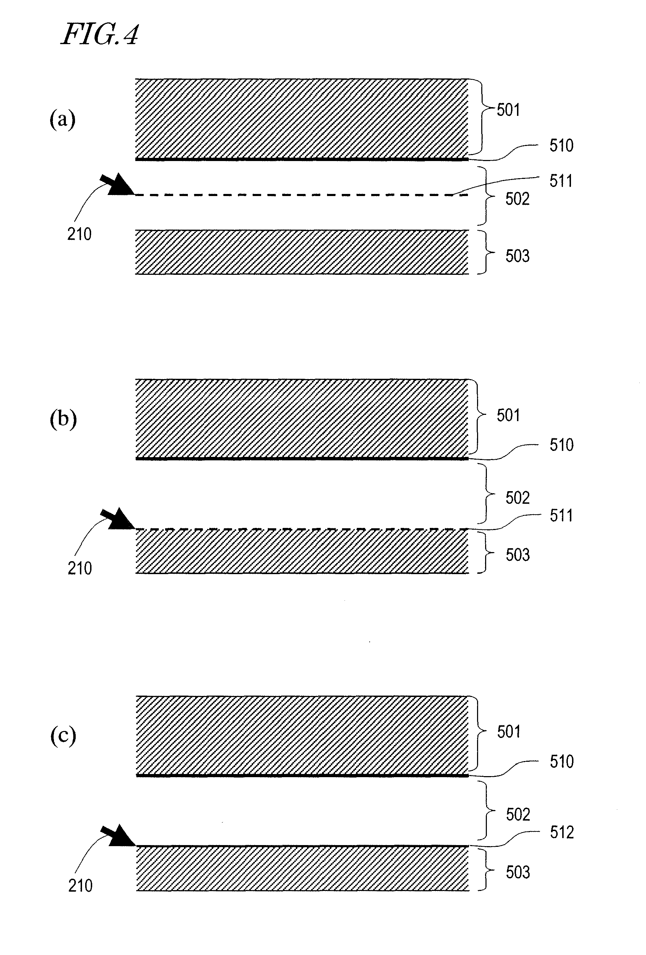

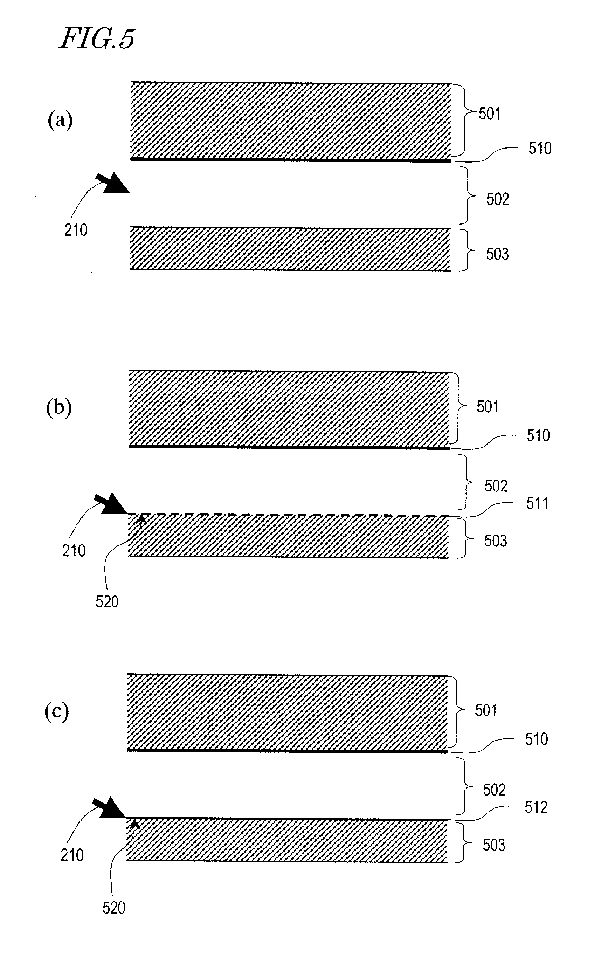

FIGS. **4(a)** through **4(c)** and FIGS. **5(a)** through **5(c)** show examples of such procedures. In these drawings, regions **501, 502** and **503** schematically represent mutually different tissues.

First, as shown in FIG. **4(a),** a borderline **510** is drawn as a first type of borderline on the boundary between the tissues **501** and **502.** This borderline **510** is obtained as a line segment that has been drawn as a trace of the cursor **210** by allowing the operator to move the cursor **210** along the boundary between the tissues **501** and **502** using the user interface **130.** By determining the borderline **510** in this manner, information about the borderline **510** is stored in the borderline storage section **151.** Alternatively, the operator may also determine the borderline **510** by specifying multiple locations with the cursor **210** and generating a line or a polygon passing all of those specified locations as described above. Still alternatively, the borderline **510** may also be determined by generating either a tropic by a minimum square method or a spline function with respect to the multiple locations specified.

After the borderline **510** has been determined in this manner, the second borderline generating section **152** generates a borderline **511,** having the same shape and the same direction as the borderline **510,** at the tip of the cursor **210** in accordance with the operator's command that has been entered through the user interface **130.** For example, by entering a "copy" command through the user interface **130,** the borderline **511** may be generated and presented on the monitor screen. This borderline **511** is generated based on the information about the borderline **510** stored in the borderline storage section **151** and the location information of the cursor **210.** When the operator moves the cursor **210,** the borderline **511** is also translated.

Next, as shown in FIG. **4(b),** the operator moves the cursor **210** to a location where a borderline should be generated. In the example shown in FIG. **4(b),** the cursor **210** is located on the boundary between the regions **502** and **503** to determine a borderline on the boundary between the regions **502** and **503.**

When the cursor **210** is moved to a particular location in this manner, the borderline **510** is translated to that location, thereby generating and displaying a borderline **511** there.

Subsequently, if the operator inputs a confirmation command such as a paste command through the user interface **130,** a borderline **512** is established at that location as shown in FIG. **4(c).** In this manner, the borderline **512,** generated by translating the borderline **510** that has been drawn by the operator, is determined as the second type of borderline.

Alternatively, another procedure may also be adopted. First, as shown in **FIG. 5(a)**, a borderline **510** is drawn as a first type of borderline on the boundary between the tissues **501** and **502.** The borderline **510** is drawn in the same way as already described with reference to FIG. **4(a).**

After the borderline storage section **151** stores information about the borderline **510** as a first type of borderline, the apparatus changes into a "copy" mode in accordance with an operator's command that has been entered through the user interface **130.** According to this procedure, the operator can move the cursor **210** to his or her desired location with the user interface **130** without displaying a copy of the borderline **510** on the screen.

As shown in FIG. **5(b),** the operator has moved the cursor **210** to the location where a borderline should be generated. In the example shown in FIG. **5(b),** the cursor **210** is located on the boundary between the regions **502** and **503** to determine a borderline on the boundary between the regions **502** and **503.** After having moved the cursor **210** to his or her desired location, the operator enters a command to generate a copy of the borderline **510** through the user interface **130.** In response, the second borderline generating section **152** generates the borderline **511** by translating the borderline **510** to the location specified with the cursor **210** in accordance with the information about the first type of borderline.

Subsequently, if the operator inputs a confirmation command such as an enter command through the user interface **130,** the borderline **512** is established at that location as shown in FIG. **5(c).** In this manner, the borderline **512,** generated by translating the borderline **510** that has been drawn by the operator, is determined.

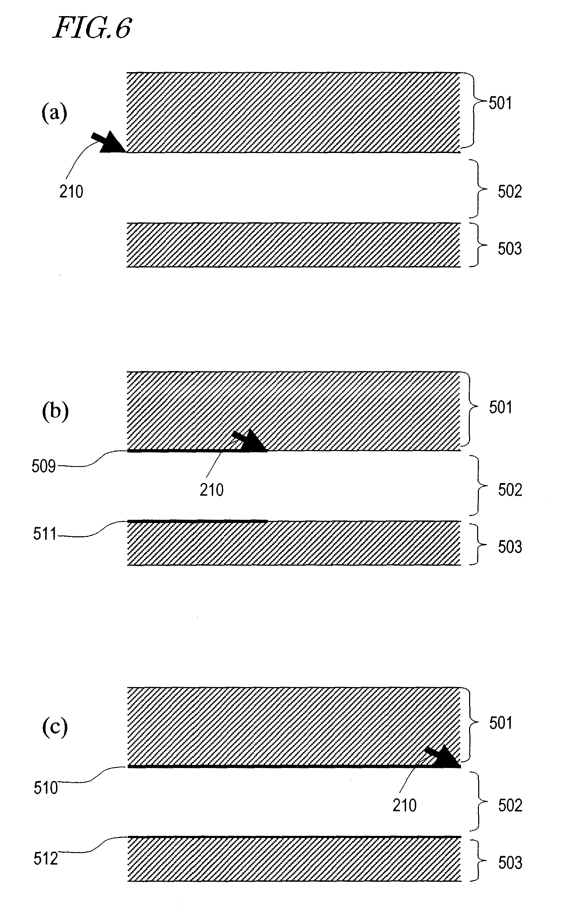

Still another procedure may also be adopted. First, as shown in FIG. **6(a),** the operator puts the cursor **210** on the boundary between the tissues **501** and **502.** Next, as shown in FIG. **6(b),** the operator traces the boundary between the tissues **501** and **502** with the cursor **210,** thereby drawing a line segment **509** indicating a first type of borderline. In the meantime, the borderline determining section **150** automatically draws a trace of points, which are located on the same acoustic line as, but at a predetermined distance from, the cursor **210,** as another line segment **511.** For that purpose, the first borderline generating section **153** gets the location of the cursor **210** and outputs information about that location to the borderline storage section **151.** The operator inputs the interval between the locations of points to be automatically generated and the cursor **210** through the user interface **130.** In the example shown in FIG. **6(b),** the borderline between the tissues **502** and **503** should be generated automatically, and therefore, the interval from the boundary between the tissues **501** and **502** to the one between the tissues **502** and **503** is defined by the operator. The second borderline generating section **152** generates the line segment **511** based on the location of the cursor **210** that has been read from the borderline storage section **151** and the interval that has been entered through the user interface **130**. Optionally, the interval may be determined in advance before the series of operations are started in the processing step shown FIG. **6(a)**.

As shown in FIG. **6(c),** when the operator finishes tracing the boundary between the tissues **501** and **502** with the cursor **210,** a borderline **510** is established as a first type of borderline. At the same time, a borderline **512** is automatically determined as a second type of borderline. This borderline **512** is a trace of points that are located at a predetermined distance from the location of the cursor **210** that draws the borderline **510,** and therefore, has been obtained by translating the borderline **510**. In this manner, a borderline **510** drawn by the operator and a borderline **512** that is a translated version of the borderline **510** are obtained.

As shown in FIG. **3,** if a blood vessel is viewed on a plane that is parallel to its axis, an anterior wall and a posterior wall are seen with a vascular lumen, where blood flows, interposed, and each of the anterior and posterior walls consists of an intima, a media and an adventitia. In a target region of measurements to be done to calculate an attribute property value of the blood vessel, the boundaries between these tissues should be substantially fixed, i.e., the diameter of the vascular lumen and the thicknesses of the respective tissues should remain approximately the same, unless some disease has occurred there. That is why the boundary between the vascular flow and the intima, the boundary between the intima and media, the boundary between the media and adventitia, and the boundary between the adventitia and a surrounding tissue would be ideally roughly parallel to each other. Even if the axis of the blood vessel is not straight, the diameter of the vascular lumen and the thicknesses of the respective tissues still remain approximately the same, and therefore, the respective boundaries can be obtained by translating one of them to another.

For that reason, the second type of borderline generated by the borderline determining section **150** will agree with the actual boundary between the tissues unless the target region is adjacent to a portion with some disease. In this manner, the burden imposed on the operator by forcing him or her to trace the entire boundary and the time it will take to determine the borderlines can be both reduced. Particularly in a situation where a boundary presented on a tomographic image is not so clear, it would not cause so much trouble to the operator to specify only one point as a borderline location but it should be a lot of trouble for the operator to trace such an indefinite boundary entirely with the cursor. According to the present invention, such a burden can be lightened.

Also, although the thicknesses of the intima and media of a vascular wall and the diameter of a blood vessel could vary significantly according to the health condition of the given subject or from one person to another, the thickness of the adventitia hardly varies. It is said that at the carotid of a healthy person, the adventitia will have a thickness of approximately 0.3 mm. For that reason, unless the adventitia has any disease, the thickness of the adventitia may be defined in advance and the boundary between the adventitia and a surrounding tissue may be determined at a location that is a predetermined thickness away from the media-adventitia boundary being determined. Then, the burden imposed on the operator can be further alleviated. The adventitia preferably has a thickness of approximately 0.2 mm to approximately 0.4 mm.

The second type of borderline determined by the borderline determining section **150** does not have to be located on the boundary between the adventitia and a surrounding tissue but may also be located on any of various other boundaries. Hereinafter, an example in which the second type of borderline is determined somewhere else will be described.

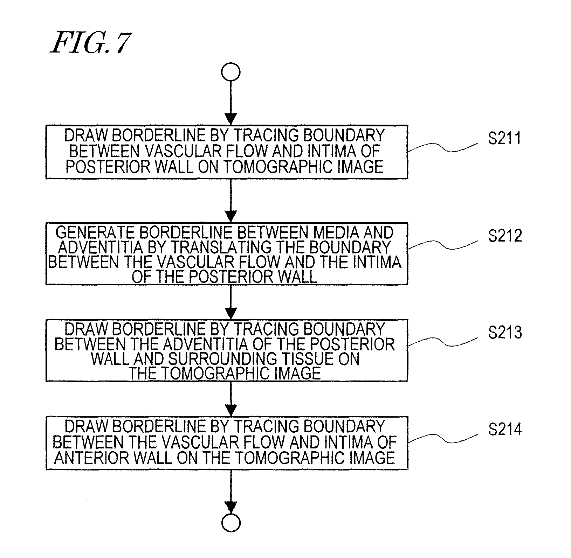

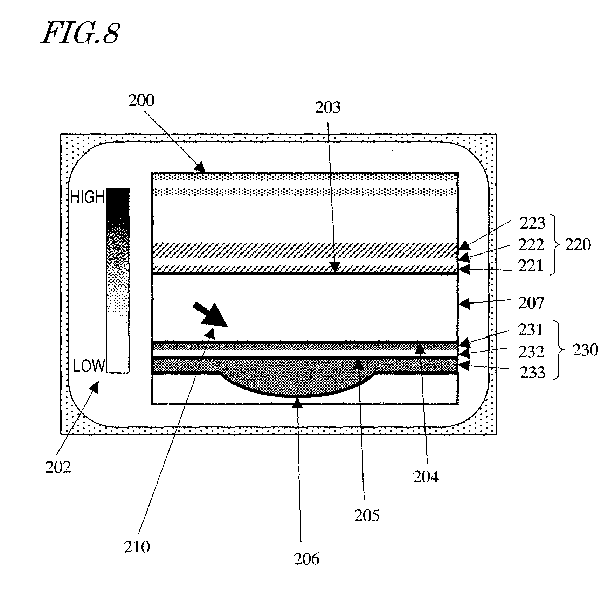

FIG. **8** illustrates a tomographic image representing a case where a boss-like disease has occurred on the outer surface of a blood vessel. FIG. **7** is a flowchart showing the procedure of determining a borderline on the tomographic image shown in FIG. **8.**

First, in Step **S211,** by moving a cursor **210,** which is displayed on the screen of the monitor **107,** using the user interface **130** such as a trackball or a mouse provided for the ultrasonic diagnostic apparatus, the boundary between the vascular flow **207** and the intima **231** of the posterior wall **230** on the tomographic image **200** is traced with the cursor **210**. In this manner, a borderline **204** is drawn between the vascular flow **207** and the intima **231** of the posterior wall **230** on the screen. The borderline storage section **151** stores information about the borderline **204** as a first type of borderline.

Next, in Step **S212,** the second borderline generating section **152** translates the borderline **204** in accordance with the information about the borderline **204** that is stored in the borderline storage section **151,** thereby generating a borderline **205** on the boundary between the media **232** and adventitia **233** of the posterior wall **230** as a second type of borderline.

Subsequently, in Step **S213,** by moving the cursor **210** on the screen of the monitor **107** using the user interface **130,** the boundary between the adventitia **233** of the posterior wall **230** and a surrounding tissue on the tomographic image **200** is traced with the cursor **210**. In this manner, a borderline **206** is drawn between the adventitia **233** of the posterior wall **230** and the surrounding tissue. The borderline storage section **151** stores information about the borderline **206** as another borderline of the first type.

Finally, in Step **S214,** a borderline **203** between the vascular flow **207** and the intima **221** of the anterior wall **220** is drawn in the same way as in Step **S213.** The borderline storage section **151** stores information about the borderline **203** as still another borderline of the first type.

By following such a procedure, the burden imposed on the operator by tracing boundaries and the time it takes to determine the borderlines can be reduced by one step.

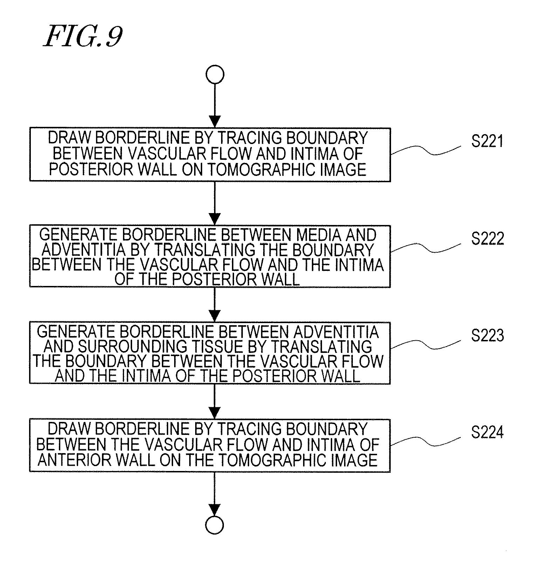

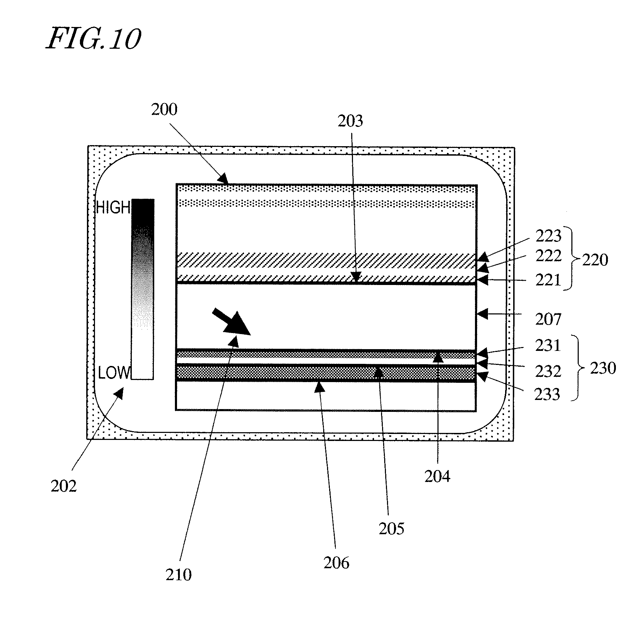

FIG. **10** illustrates a tomographic image of a healthy person's blood vessel. FIG. **9** is a flowchart showing a procedure for determining borderlines on the tomographic image shown in FIG. **10.**

First, in Step **S221,** by moving a cursor **210,** which is displayed on the screen of the monitor **107,** using the user interface **130** such as a trackball or a mouse provided for the ultrasonic diagnostic apparatus, the boundary between the vascular flow **207** and the intima **231** of the posterior wall **230** on the tomographic image **200** is traced with the cursor **210.** In this manner, a borderline **204** is drawn between the vascular flow **207** and the intima **231** of the posterior wall **230** on the screen. The borderline storage section **151** stores information about the borderline **204** as a first type of borderline.

Next, in Step **S222,** the second borderline generating section **152** translates the borderline **204** in accordance with the information about the borderline **204** that is stored in the borderline storage section **151,** thereby generating a borderline **205** on the boundary between the media **232** and adventitia **233** of the posterior wall **230** as a second type of borderline.

Subsequently, in Step **S223,** the borderline **204** is translated again in accordance with the location information of the borderline **204** that is stored in the borderline storage section **151,** thereby generating a borderline **206** on the boundary between the adventitia **233** of the posterior wall **230** and its surrounding tissue.

Thereafter, in Step **S224,** by moving the cursor **210** on the screen of the monitor **107** using the user interface **130,** the boundary between the vascular flow **207** and the intima **221** of the anterior wall **220** on the tomographic image **200** is traced with the cursor **210.** In this manner, a borderline **203** is drawn between the vascular flow **207** and the intima **221** of the anterior wall **220.**

By following such a procedure, the burden imposed on the operator by tracing boundaries and the time it takes to determine the borderlines can be reduced by two steps.

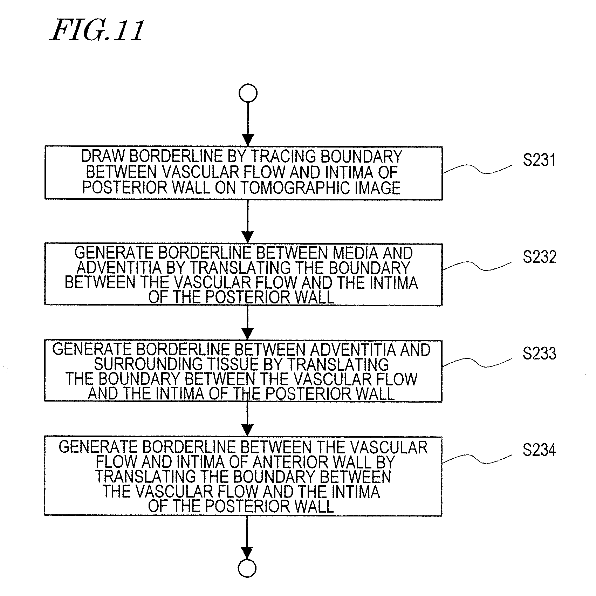

FIG. **11** is a flowchart showing another procedure for determining borderlines on the tomographic image shown in FIG. **10.**

First, in Step **S231,** by moving a cursor **210,** which is displayed on the screen of the monitor **107,** using the user interface **130** such as a trackball or a mouse provided for the ultrasonic diagnostic apparatus, the boundary between the vascular flow **207** and the intima **231** of the posterior wall **230** on the tomographic image **200** is traced with the cursor **210.** In this manner, a borderline **204** is drawn between the vascular flow **207** and the intima **231** of the posterior wall **230** on the screen. The borderline storage section **151** stores information about the borderline **204** as a first type of borderline.

Next, in Step **S232,** the second borderline generating section **152** translates the borderline **204** in accordance with the information about the borderline **204** that is stored in the borderline storage section **151,** thereby generating a borderline **205** on the boundary between the media **232** and adventitia **233** of the posterior wall **230** as a second type of borderline.

Subsequently, in Step **S223,** the second borderline generating section **152** translates the borderline **204** again in accordance with the location information of the borderline **204** that is stored in the borderline storage section **151,** thereby generating a borderline **206** on the boundary between the adventitia **233** of the posterior wall **230** and its surrounding tissue as a second type of borderline.

In the same way, in Step **S234,** the borderline **204** is translated once again in accordance with the location information of the borderline **204** that is stored in the borderline storage section **151,** thereby generating a borderline **203** on the boundary between the vascular flow **207** and the intima **221** of the anterior wall **220** as another borderline of the second type.

By following such a procedure, the burden imposed on the operator by tracing boundaries and the time it takes to determine the borderlines can be reduced by three steps.

As described above, by translating a borderline, the burden imposed on the operator to determine at least one borderline and the time it takes to determine that borderline can be reduced.

Hereinafter, it will be described with reference to FIGS. **1(a)** and **1(b)** how to measure an IMT with the ultrasonic diagnostic apparatus of the present invention. First, ultrasonic waves are transmitted and received with the probe **101,** thereby obtaining a series of tomographic images of a vascular wall, which are viewed on a plane parallel to its axis and which are generated consecutively at multiple points in time, and presenting them on the monitor **107** in real time. Meanwhile, the tomographic image data is stored in the memory **120.** Next, in the freeze state, the data is read from the memory **120** and the best tomographic image to measure the IMT is presented on the monitor **107.**

By manipulating the user interface **130,** the operator moves the cursor **210** that is displayed on the screen of the monitor **107** and traces the boundary between the vascular flow and the intima of the anterior or posterior wall on the tomographic image on the monitor **107** with the cursor **210,** thereby drawing a first type of borderline. The borderline storage section **151** stores information about this borderline of the first type.

Next, if the boundary between the media and adventitia is parallel to the one between the vascular flow and the intima (e.g., if there is no diseased part in the intima or in the media), then the second borderline generating section **152** translates the borderline that has been drawn on the boundary between the vascular flow and the intima, thereby generating a second type of borderline between the media and adventitia at a location corresponding to the boundary between the media and adventitia. On the other hand, if these two boundaries are not parallel to each other (e.g., if there is any diseased part in the intima), the borderline between the media and adventitia is also determined in the same way. That is to say, using the user interface **130,** another borderline of the first type is drawn by moving the cursor **210** on the screen of the monitor **107** and tracing the boundary between the media and adventitia on the tomographic image with the cursor **210.** Also, if the anterior and posterior walls are parallel to each other on the tomographic image, then the second borderline generating section **152** may translate the borderline that has been determined on the anterior or posterior wall, thereby generating a borderline indicating the boundary between the vascular flow and the intima of the anterior or posterior wall or between the media and adventitia thereof. Optionally, the apparatus may also be designed so as to allow the operator to determine, with the user interface **130,** whether a new borderline should be generated by getting the borderline that has been drawn by the operator translated by the second borderline generating section **152** or should be generated by himself or herself using the user interface **130.** The IMT can be calculated based on the interval from the borderline between the vascular flow and the intima to the one between the media and adventitia thus obtained. By adopting such a procedure, the burden and the time-consuming job imposed on the operator by tracing boundaries can be alleviated. In addition, the IMT can be calculated in a shorter time, too.

Hereinafter, it will be described how to calculate the elasticities. First, ultrasonic waves are transmitted and received with the probe **101,** thereby obtaining a series of tomographic images of a vascular wall, which are viewed on a plane parallel to its axis and which are generated consecutively at multiple points in time, and presenting them on the monitor **107** in real time. Meanwhile, the tissue tracking section **105** tracks the vascular wall tissue. Using the blood pressure values provided by the blood pressure manometer **111,** the elasticity calculating section **108** calculates radial elasticities and gets an image representing the distribution of the radial elasticities presented on the monitor **107.** The tomographic image data and the image data representing the distribution of the radial elasticities are stored in the memories **120** and **121,** respectively. The tomographic images are presented consecutively, while the image representing the distribution of the elasticities is updated once in every cardiac cycle. After having changed the modes of the ultrasonic diagnostic apparatus into the freeze state, the data is read from the memory **121** and the best image representing the distribution of the elasticities and a tomographic image synchronized with that image are read from the memory **120** and presented on the monitor **107.**

By manipulating the user interface **130,** the operator moves the cursor **210** that is displayed on the screen of the monitor **107** and traces the boundary between the vascular flow and the intima of the posterior wall on the tomographic image on the monitor **107** with the cursor **210,** thereby drawing a first type of borderline. The borderline storage section **151** stores information about the first type of borderline thus determined.

Next, if the boundary between the adventitia of the posterior wall and its surrounding tissue is parallel to the one between the vascular flow and the intima (e.g., if there is no diseased part in the intima or in the adventitia), then the second borderline generating section **152** translates the first type of borderline that has been drawn on the boundary between the vascular flow and the intima, thereby generating a second type of borderline between the adventitia and the surrounding tissue at a location corresponding to the boundary between the adventitia and the surrounding tissue. On the other hand, if these two boundaries are not parallel to each other (e.g., if there is any diseased part in the intima or in the adventitia), the borderline between the adventitia and the surrounding tissue is also determined in the same way. That is to say, using the user interface **130,** another borderline of the first type is drawn by moving the cursor **210** on the screen of the monitor **107** and tracing the boundary between the adventitia and the surrounding tissue on the tomographic image with the cursor **210.**

Next, if the boundary between the vascular flow and the intima of the anterior wall is parallel to the one between the vascular flow and the intima of the posterior wall, then the second borderline generating section **152** translates the first type of borderline that has been drawn on the boundary between the vascular flow and the intima of the posterior wall, thereby generating a second type of borderline at a location corresponding to the boundary between the vascular flow and the intima of the anterior wall. On the other hand, if these two boundaries are not parallel to each other, the borderline between the vascular flow and the intima of the anterior wall is also determined in the same way. That is to say, using the user interface **130,** another borderline of the first type is drawn by moving the cursor **210** on the screen of the monitor **107** and tracing the boundary between the vascular flow and the intima of the anterior wall on the tomographic image with the cursor **210.**

Based on the interval from the borderline between the vascular flow and the intima of the posterior wall to the one between the adventitia and the surrounding tissue thus obtained, the initial radial thickness **h0** of the overall blood vessel can be calculated. Based on the interval from the borderline between the vascular flow and the intima of the anterior wall to the one between the vascular flow and the intima of the posterior wall thus obtained, the initial radius **r0** of the blood vessel can be calculated. And the circumferential elasticity can be calculated based on these values. By adopting such a procedure, the burden and the time-consuming job imposed on the operator by tracing boundaries can be alleviated. In addition, the circumferential elasticity can be calculated in a shorter time, too.

In the preferred embodiments described above, the first borderline generating section generates a line segment or a polygon as the first type of borderline by allowing the operator to draw it with the user interface **130** or specify multiple points. However, the first type of borderline may also be generated automatically. For example, a vascular flow portion and a vascular wall portion of a subject have significantly different acoustic impedances and the image signals thereof have different amplitudes. That is why the boundary between the vascular flow portion and the vascular wall portion, i.e., the boundary between the vascular flow and the intima of the vascular wall, can be detected automatically based on the amplitudes of the image signals. The first borderline generating section may detect the boundary between the vascular flow and the intima of the anterior or posterior wall based on the image signals generated by the tomographic image processing section and may determine the first type of borderline on that boundary. Also, the vascular flow portion would produce a Doppler effect in the reflected echo due to the flow of the blood. For that reason, the first borderline generating section may also detect the boundary between the vascular flow and the intima of the anterior or posterior wall by utilizing a Doppler mode and determine the first type of borderline on the boundary detected.

Meanwhile, the acoustic impedances of the intima, media and adventitia included in a vascular wall are not significantly different from each other. For that reason, it could sometimes be difficult to detect them by any of these methods. However, as described above, the boundaries between these tissues in the vascular wall are often parallel to the boundary between the vascular flow and the intima unless there is any diseased part there. In that case, if the operator can specify the boundary between the media and adventitia at an appropriate location by looking at the tomographic image, then the second borderline generating section can translate the first type of borderline, which has been automatically detected and determined, to the location specified by the operator and generate a second type of borderline there. Consequently, the burden imposed on the operator can be lightened significantly and the variations caused by the operators' decisions can be reduced greatly.

## INDUSTRIAL APPLICABILITY

The present invention is effectively applicable to an ultrasonic diagnostic apparatus that estimates the shape or attribute property value of the vascular wall of a subject.

## Claims

1. An ultrasonic diagnostic apparatus for measuring a vascular wall, the apparatus comprising:

    a transmitting section that drives a probe to transmit an ultrasonic wave toward a subject including a blood vessel;
    a receiving section that receives an ultrasonic echo, produced by getting the ultrasonic wave reflected by the subject, at the probe to generate a received signal;
    a tomographic image processing section for generating an image signal based on the received signal to present the tomographic image of the subject on a display section;
    a first borderline generating section for determining a first type of borderline based on the tomographic image presented on the display section; and
    a second borderline generating section for generating at least one borderline of a second type by translating the first type of borderline.

2. The ultrasonic diagnostic apparatus of claim 1, further comprising a user interface that allows an operator to specify his or her desired location on the tomographic image.

3. The ultrasonic diagnostic apparatus of claim 2, wherein the borderline generating section generates the second type of borderline by translating the first type of borderline to the location that has been specified by the operator with the user interface in accordance with information about the first type of borderline.

4. The ultrasonic diagnostic apparatus of claim 3, wherein the first borderline generating section stores the information about the first type of borderline that has been drawn by the operator with the user interface.

5. The ultrasonic diagnostic apparatus of claim 4, wherein the first borderline generating section generates either a line segment or a polygon as the first type of borderline at the location that has been specified by the operator.

6. The ultrasonic diagnostic apparatus of claim 4, wherein the first borderline generating section stores, as information about the first type of borderline, information about a line segment that has been drawn by the operator on the tomographic image with the user interface.

7. The ultrasonic diagnostic apparatus of claim 3, wherein the first type of borderline is located on at least one boundary that is selected from a group of boundaries consisting of boundaries between the intima of the anterior and posterior walls of the blood vessel and vascular flow, boundaries between the media and adventitia of the anterior and posterior walls of the blood vessel, and boundaries between the adventitia of the anterior and posterior walls of the blood vessel and their surrounding tissue, and wherein the second type of borderline is located on at least another one of the group of boundaries.

8. The ultrasonic diagnostic apparatus of claim 7, wherein the first borderline generating section detects the boundary between the intima of the anterior or posterior wall of the blood vessel and the vascular flow based on the image signal and determines the first type of borderline on the boundary detected.

9. The ultrasonic diagnostic apparatus of claim 8, wherein the first type of borderline is located on the boundary between the intima of the anterior or posterior wall and the vascular flow and the second type of borderline is located on the boundary between the media and adventitia thereof, and
wherein the intima-media thickness of the vascular wall is calculated by reference to the first and second types of borderlines.

10. The ultrasonic diagnostic apparatus of claim 9, further comprising:

   a tissue tracking section for tracking the motion of a tissue of the subject based on the received signal; and
   an attribute value calculating section that receives information about the blood pressure of the subject and calculates an attribute value of the tissue based on the motion of the tissue tracked,

   wherein the attribute value of at least one tissue, which is selected from the group consisting of the intima, media and adventitia of the blood vessel, is calculated with respect to the first and second types of borderlines.

11. The ultrasonic diagnostic apparatus of claim 10, wherein the first type of borderline is located on the boundary between the intima and the vascular flow and the second type of borderline is located between the adventitia and the surrounding tissue, and
wherein by calculating the radius of the blood vessel and the thickness of the vascular wall by reference to the first and second types of borderlines, a circumferential elasticity is calculated as the attribute value.

12. A method for determining the boundaries of a vascular wall using an ultrasonic diagnostic apparatus, the method comprising the step of:

   generating a second type of borderline by translating a first type of borderline on a tomographic image of a subject's blood vessel that has been produced by transmitting and receiving an ultrasonic wave, the first type of borderline being determined so as to be located on at least one boundary that is selected from a group of boundaries consisting of boundaries between the intima of the anterior and posterior walls of the blood vessel and vascular flow, boundaries between the media and adventitia of the anterior and posterior walls of the blood vessel, and boundaries between the adventitia of the anterior and posterior walls of the blood vessel and their surrounding tissue, the second type of borderline being located on at least another one of the group of boundaries.

13. The method of claim 12, further comprising the step of storing, as information about the first type of borderline, information about a line segment that has been drawn on the tomographic image by the operator using a user interface,
wherein the step of generating the second type of borderline includes generating the second type of borderline based on the stored information about the first type of borderline.

14. The method of claim 12, further comprising the step of generating either a line segment or a polygon as the first type of borderline at the location that has been specified on the tomographic image of the blood vessel by the operator using the user interface.

15. The method of claim 12, further comprising the step of detecting the boundary between the intima of the anterior or

EP 1 961 384 A1

posterior wall of the blood vessel and the vascular flow based on an image signal representing the tomographic image and determining the first type of borderline on the boundary detected.

14

*FIG.1*

(a)

```
                                              120                  107
         102                                  ┌──────────┐    ┌──────────┐
    ┌──────────┐                              │  MEMORY  │    │ MONITOR  │
    │ TRANS-   │                              └──────────┘    └──────────┘
101 │ MITTING  │   103        104                          106
┌────────┐ │ SECTION │ ┌──────────────┐              ┌──────────────┐
│ PROBE  │ └──────────┘ │ TOMOGRAPHIC  │              │   IMAGE      │
└────────┘ ┌──────────┐ │IMAGE PROCESSING│            │ SYNTHESIZING │
           │RECEIVING │ │   SECTION    │              │   SECTION    │
           │ SECTION  │ └──────────────┘              └──────────────┘
           └──────────┘
```

100        130                    105                          108
┌──────────┐  ┌──────────┐    ┌──────────┐              ┌──────────────┐
│ CONTROL  │←─│    UI    │    │  TISSUE  │              │  ELASTICITY  │
│ SECTION  │  └──────────┘    │ TRACKING │              │ CALCULATING  │
└──────────┘                  │ SECTION  │              │   SECTION    │
                              └──────────┘              └──────────────┘

                                              121
111                                       ┌──────────┐
┌──────────┐                              │  MEMORY  │
│  BLOOD   │                              └──────────┘
│ PRESSURE │
│MANOMETER │
└──────────┘

(b)

```
                                   130
        107                    ┌──────────┐
    ┌──────────┐               │    UI    │
    │ MONITOR  │               └──────────┘        150
    └──────────┘                              ┌──────────────────────┐
104              106                          │ FIRST BORDERLINE     │ 153
┌──────────────┐ ┌──────────────┐             │ GENERATING SECTION   │
│ TOMOGRAPHIC  │ │   IMAGE      │             ├──────────────────────┤
│IMAGE PROCESSING│→│ SYNTHESIZING │←──────────│ SECOND BORDERLINE    │ 152
│   SECTION    │ │   SECTION    │             │ GENERATING SECTION   │
└──────────────┘ └──────────────┘             ├──────────────────────┤
                      108                     │ BORDERLINE           │ 151
                 ┌──────────────┐             │ STORAGE SECTION      │
                 │  ELASTICITY  │             └──────────────────────┘
                 │ CALCULATING  │
                 │   SECTION    │
                 └──────────────┘
```

## FIG.2

DRAW BORDERLINE BY TRACING BOUNDARY
BETWEEN VASCULAR FLOW AND INTIMA OF
POSTERIOR WALL ON TOMOGRAPHIC IMAGE — S201

DRAW BORDERLINE BY TRACING BOUNDARY
BETWEEN MEDIA AND ADVENTITIA OF
THE POSTERIOR WALL ON THE TOMOGRAPHIC IMAGE — S202

GENERATE BORDERLINE BETWEEN THE ADVENTITIA
AND SURROUNDING TISSUE BY TRANSLATING
THE MEDIA-ADVENTITIA BORDERLINE
OF THE POSTERIOR WALL — S203

DRAW BORDERLINE BY TRACING BOUNDARY
BETWEEN THE VASCULAR FLOW AND INTIMA OF
ANTERIOR WALL ON THE TOMOGRAPHIC IMAGE — S204

# FIG.3

## FIG.4

(a)

501
510
511
502
210
503

(b)

501
510
502
511
210
503

(c)

501
510
502
512
210
503

## FIG.5

(a)

210

501
510
502
503

(b)

210

520

501
510
502
511
503

(c)

210

520

501
510
502
512
503

# FIG.6

(a)

210
501
502
503

(b)

509
511
210
501
502
503

(c)

510
512
210
501
502
503

## FIG.7

```
                    ○
                    │
                    ▼
┌─────────────────────────────────────────┐
│ DRAW BORDERLINE BY TRACING BOUNDARY      │
│ BETWEEN VASCULAR FLOW AND INTIMA OF      │ ～ S211
│ POSTERIOR WALL ON TOMOGRAPHIC IMAGE      │
└─────────────────────────────────────────┘
                    │
                    ▼
┌─────────────────────────────────────────┐
│ GENERATE BORDERLINE BETWEEN MEDIA AND    │
│ ADVENTITIA BY TRANSLATING THE BOUNDARY   │ ～ S212
│ BETWEEN THE VASCULAR FLOW AND THE INTIMA │
│ OF THE POSTERIOR WALL                    │
└─────────────────────────────────────────┘
                    │
                    ▼
┌─────────────────────────────────────────┐
│ DRAW BORDERLINE BY TRACING BOUNDARY      │
│ BETWEEN THE ADVENTITIA OF THE POSTERIOR  │ ～ S213
│ WALL AND SURROUNDING TISSUE ON           │
│ THE TOMOGRAPHIC IMAGE                    │
└─────────────────────────────────────────┘
                    │
                    ▼
┌─────────────────────────────────────────┐
│ DRAW BORDERLINE BY TRACING BOUNDARY      │
│ BETWEEN THE VASCULAR FLOW AND INTIMA OF  │ ～ S214
│ ANTERIOR WALL ON THE TOMOGRAPHIC IMAGE   │
└─────────────────────────────────────────┘
                    │
                    ▼
                    ○
```

EP 1 961 384 A1

FIG.8

22

## FIG.9

DRAW BORDERLINE BY TRACING BOUNDARY BETWEEN VASCULAR FLOW AND INTIMA OF POSTERIOR WALL ON TOMOGRAPHIC IMAGE — S221

GENERATE BORDERLINE BETWEEN MEDIA AND ADVENTITIA BY TRANSLATING THE BOUNDARY BETWEEN THE VASCULAR FLOW AND THE INTIMA OF THE POSTERIOR WALL — S222

GENERATE BORDERLINE BETWEEN ADVENTITIA AND SURROUNDING TISSUE BY TRANSLATING THE BOUNDARY BETWEEN THE VASCULAR FLOW AND THE INTIMA OF THE POSTERIOR WALL — S223

DRAW BORDERLINE BY TRACING BOUNDARY BETWEEN THE VASCULAR FLOW AND INTIMA OF ANTERIOR WALL ON THE TOMOGRAPHIC IMAGE — S224

## FIG.10

## FIG.11

```
        ○
        │
        ▼
┌─────────────────────────────────┐
│  DRAW BORDERLINE BY TRACING BOUNDARY │     ⌇  S231
│  BETWEEN VASCULAR FLOW AND INTIMA OF │
│  POSTERIOR WALL ON TOMOGRAPHIC IMAGE │
└─────────────────────────────────┘
        │
        ▼
┌─────────────────────────────────┐
│  GENERATE BORDERLINE BETWEEN MEDIA AND │     ⌇  S232
│  ADVENTITIA BY TRANSLATING THE BOUNDARY │
│  BETWEEN THE VASCULAR FLOW AND THE INTIMA │
│        OF THE POSTERIOR WALL │
└─────────────────────────────────┘
        │
        ▼
┌─────────────────────────────────┐
│  GENERATE BORDERLINE BETWEEN ADVENTITIA AND │   ⌇  S233
│    SURROUNDING TISSUE BY TRANSLATING │
│  THE BOUNDARY BETWEEN THE VASCULAR FLOW │
│   AND THE INTIMA OF THE POSTERIOR WALL │
└─────────────────────────────────┘
        │
        ▼
┌─────────────────────────────────┐
│  GENERATE BORDERLINE BETWEEN THE VASCULAR │   ⌇  S234
│   FLOW AND INTIMA OF ANTERIOR WALL BY │
│   TRANSLATING THE BOUNDARY BETWEEN │
│    THE VASCULAR FLOW AND THE INTIMA │
│        OF THE POSTERIOR WALL │
└─────────────────────────────────┘
        │
        ▼
        ○
```

*FIG.12*

## FIG.13

(a)

(b)

R WAVE

t [sec]

ELECTROCARDIOGRAPHIC
COMPLEX ECG

TRACKING WAVEFORM TA
AT MEASURING POINT A

TRACKING WAVEFORM TB
AT MEASURING POINT B

THICKNESS VARIATION
WAVEFORM
W=TB-TA

ΔW

# FIG.14

**EP 1 961 384 A1**

| INTERNATIONAL SEARCH REPORT | International application No. |
|---|---|
| | PCT/JP2006/324855 |

A. CLASSIFICATION OF SUBJECT MATTER
A61B8/08(2006.01)i

According to International Patent Classification (IPC) or to both national classification and IPC

B. FIELDS SEARCHED

Minimum documentation searched (classification system followed by classification symbols)
A61B8/08

Documentation searched other than minimum documentation to the extent that such documents are included in the fields searched
Jitsuyo Shinan Koho          1922-1996   Jitsuyo Shinan Toroku Koho   1996-2007
Kokai Jitsuyo Shinan Koho    1971-2007   Toroku Jitsuyo Shinan Koho   1994-2007

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 2004-305236 A  (Shimadzu Corp.),<br>04 November, 2004 (04.11.04),<br>Par. No. [0019]; Fig. 2<br>(Family: none) | 1-15 |
| A | JP 11-197152 A  (Toshiba Corp.),<br>27 July, 1999 (27.07.99),<br>Par. No. [0025]<br>(Family: none) | 1-15 |

☐ Further documents are listed in the continuation of Box C.   ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 13 February, 2007 (13.02.07) | 20 February, 2007 (20.02.07) |

| Name and mailing address of the ISA/ | Authorized officer |
|---|---|
| Japanese Patent Office | |
| Facsimile No. | Telephone No. |

Form PCT/ISA/210 (second sheet) (April 2005)

29

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000229078 A

- JP 10005226 A

**Non-patent literature cited in the description**

- **HIROSHI FURUHATA.** Carotid Echo. Vector Core Inc, 2004

- **HIDEYUKI HASEGAWA ; HIROSHI KANAI et al.** Evaluation of regional elastic modulus of cylindrical shell with non-uniform wall thickness. *J. Med. Ultrasonics,* 2004, vol. 28 (1), J3-J13